# EUROPEAN PATENT SPECIFICATION

(11) **EP 1 836 979 B1**
(45) Date of publication and mention of the grant of the patent: **23.05.2012**
(21) Application number: 07251156.1
(22) Date of filing: 20.03.2007
(51) Int. Cl.: A61B 17/28

(54) **Medical instrument having an engagement mechanism**
Medizinisches Instrument mit Kupplungsmechanismus
Instrument médical doté d'un système embrayage

(30) Priority: 21.03.2006 US 385540
(43) Date of publication of application: 26.09.2007
(73) Proprietor: ETHICON ENDO-SURGERY, INC., Cincinnati, Ohio 45242 (US)
(72) Inventor: Measamer, John P., Cincinnati, OH 45249 (US); Coe, Jonathan Allen, Cincinnati, OH 45236 (US); Schwemberger, Richard F., Cincinnati, OH 452470 (US)
(74) Representative: Tunstall, Christopher Stephen

(56) References cited:
- EP-A- 1 518 500
- WO-A-97/08996
- DE-A1- 19 751 731
- US-A1- 2003 009 441
- US-B1- 6 599 295

## Description

**Field of the Invention**

**Background of the Invention**

The present application relates to medical instruments and, more particularly, to surgical endoscopic instruments.

Physicians use long, flexible instruments inserted through flexible endoscopes to perform certain medical procedures through natural orifices of the patient's body, sometimes preventing more costly and painful surgical procedures. A flexible endoscopic instrument may have an endoscopic shaft portion that is about 1 to about 2 meters long and about 2 to about 2 millimeters in diameter. The shaft may include a flexible tube having a channel and an actuating element such as a metallic wire extending through the channel. A control apparatus may be operatively connected to the proximal end of the actuating element for operating an end effector on the distal end of the shaft.

Occasionally unintentional, relative movement between the actuating element and the tube may result in an uninitiated action of the end effector.
This may occur, for example, while the user control is held or locked in a position and the shaft is advanced along the tortuous path of the upper or lower gastrointestinal tract of the patient. Bending of the shaft may result in a change of the effective length of the channel, thereby resulting in the relative movement between the tube and the actuating element and, in turn, causing the uninitiated action. For some instruments, such as grasping instruments, this uninitiated action may be relatively harmless, although a nuisance. For other instruments, such as clip appliers and cutting instruments, this uninitiated action could injure the patient and/or cause delays in the procedure. If the user control is not held or locked in a position and the instrument is permitted to "float" to accommodate for this relative motion, the amount of usable input motion or stroke of the user control could be diminished such that the instrument would be nonfunctional.

In addition to the issue of relative movement of shaft components during usage of some current medical instruments, there may also be an associated manufacturing issue. Sometimes it may be difficult to maintain sufficiently tight tolerances for the length and other dimensions of the shaft components to minimize process control variations that may occur during the assembly of a high volume of instruments. To avoid rejecting a high number of components, one common practice is to match components and/or custom assemble each instrument such that the user control has a full range of input motion to operate the end effector. However, when thousands of instruments are produced in this manner over a period of time, there may be a significant cost associated with manufacturing time and materials.

Accordingly, there is a need for an improved medical instrument having a shaft containing an actuating element that operatively connects a user control to an end effector such that there is no uninitiated action of the end effector or loss of user control input motion due to flexure of the instrument shaft during usage of the instrument. In addition, there is a need for an instrument that may be less costly to manufacture than conventional instruments designed for similar medical applications.

Features of the present invention that are known from EP 1 518 500 have been placed in the preamble of claim 1 appended hereto.

**Summary of the Invention**

The present invention provides a medical instrument as claimed in the appendent claims.

In one embodiment, a medical instrument may include a shaft having a channel extending therethrough and an actuating element extending through the channel. The instrument may further include an end effector disposed on the distal end of the shaft and operatively connected to the distal end of the actuating element, wherein the actuating element is adapted to move the end effector between a first configuration and a second configuration. The instrument may further include a control moveable between a first position corresponding to the first configuration, and a second position corresponding to the second configuration, wherein the control is operatively disconnected from the actuating element when the control is in the first position.
In another embodiment, a medical instrument may include a shaft having a channel extending therethrough and an actuating element extending through the channel. The instrument may further include an end effector disposed on the distal end of the shaft and operatively connected to the distal end of the actuating element, wherein the actuating element is adapted to move the end effector between a first configuration and a second configuration. The instrument may further include a handle attached to the proximal end of the shaft. The handle may have a control moveable between a first position corresponding to the first configuration, and a second position corresponding to the second configuration, wherein the control is operatively disconnected from the actuating element when the control is in the first position. The handle may also have an engagement mechanism for operatively connecting the control to the proximal end of the actuating element when the control is moved from the first position.

Other embodiments of the medical instrument will become apparent from the following description, the accompanying drawings and the appended claims.

### Brief Description of the Figures

FIGURE 1 is a side view of an exemplary embodiment of a flexible endoscopic instrument, shown while a user control is in a second position and an end effector is in a closed configuration;

FIGURE 2 is a sectional view of the distal portion of the endoscopic instrument of Fig. 1, shown with the end effector in an opened configuration;

FIGURE 3 is a side view of the distal portion of the endoscopic instrument of Fig. 1, shown with the end effector in the closed configuration;

FIGURE 4 is a cut-away view of a handle of the instrument of Fig. 1, including a first embodiment of an engagement mechanism in a first position;

FIGURE 5 is a cross-sectional view taken at line 5-5 of the engagement mechanism of Fig. 4;

FIGURE 6 is a cut-away view of the engagement mechanism shown in Fig. 4 in a second position;

FIGURE 7 is a cut-away view of the handle of the instrument of Fig. 1, including a second embodiment of an engagement mechanism while the user control is in a first position;

FIGURE 8 is a cross-sectional view taken at line 8-8 of the engagement mechanism of Fig. 7;

FIGURE 9 is a cut-away view of the a the engagement mechanism of Fig. 7 in a second position;

FIGURE 10 is a cut-away view of the handle of the instrument of Fig. 1, including a third embodiment of an engagement mechanism in a first position;

FIGURE 11 is a cut-away view of the engagement mechanism of Fig. 10 in a second position;

FIGURE 12 is a cut-away view of the handle of the instrument of Fig. 1, including a fourth embodiment of an engagement mechanism in a first position; and

FIGURE 13 is a cut-away view of the engagement mechanism of in Fig. 12 in a second position.

**Detailed Description of the Invention**

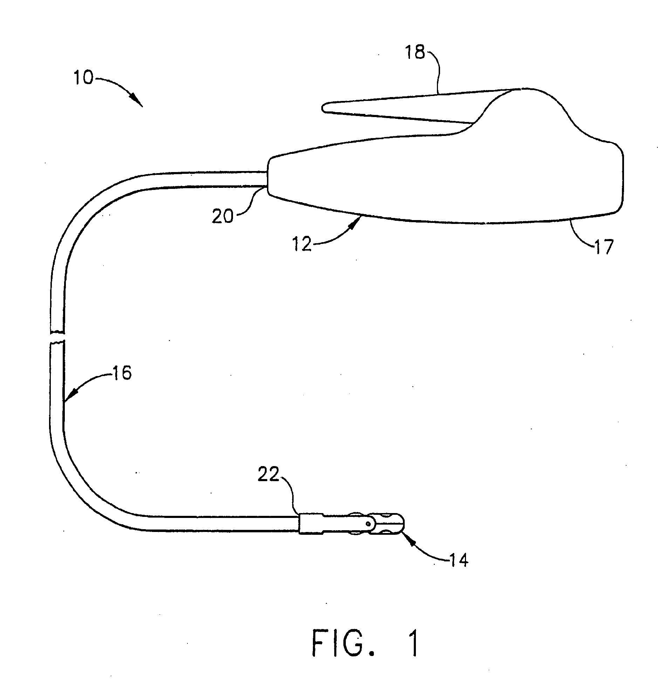

Fig. 1 is a side view of an exemplary embodiment of a flexible endoscopic instrument, generally designated 10, shown while a user control 18 is in a second position and an end effector 14 is in a closed configuration. Instrument 10 includes an elongated, flexible shaft 16 having a proximal end 20 and a distal end 22. A physician may introduce the flexible portion of instrument 10 into a lumen such as a working channel of an endoscope or a natural orifice of a patient. Instrument 10 may further include a handle 12 attached to proximal end 20 of shaft 16. A user may hold a grip 17 of handle 12 while actuating user control 18 to operate end effector 14. The configuration of handle 12 shown in Fig. 1 is provided merely as one example of many possible configurations.

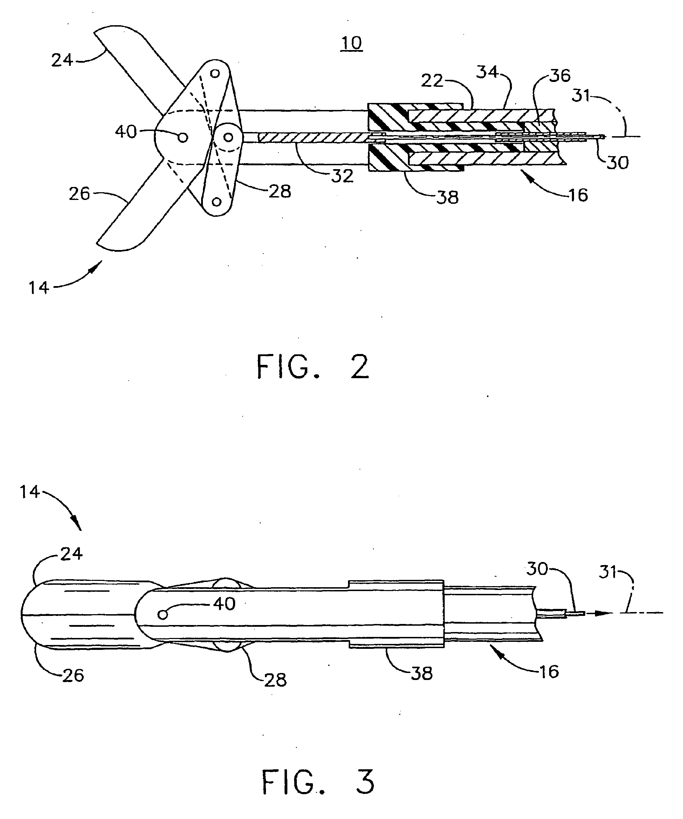

Fig. 2 is a detailed, sectional view of end effector 14 of instrument 10, shown in an opened configuration. Fig. 3 is a detailed view of end effector 14 shown in a closed configuration. End effector 14 includes a pair of opposable jaws 24, 26 operatively connected at a pivot 40 to a retainer 38, which is attached to the distal end 22 of shaft 16. Shaft 16 has a tube 34 defining a channel 36 that retains a cable 30, which serves as an actuating element. The distal end of cable 30 is operatively connected to a linkage 28 by a connector 32, such that when tension is applied to cable 30, jaws 24, 26 move to the closed position. Cable 30 may be a single filament, a multi-stranded filament or a braided filament, for example, formed from a polymer, metal or other suitable material, as is well-known in the art. Cable 30 may be flexible yet sufficiently stiff to transmit a linear force in both longitudinal directions when properly constrained in shaft 16 and grip 17.

End effector 14 is representative of many types of end effectors that may be adapted to instrument 10. Other types of end effectors may be operatively connected to cable 30 (Fig. 2) such that, when a tensile or compressive force is applied to cable 30, the end effector performs a desired action. This desired action may include, but is not limited to, the opening and closing of jaws to grasp, pinch, dissect, clamp or scissor tissue, deploying a fastening element, deploying a clip, cutting tissue, deploying staples, injecting matter into tissue, deploying a stent or depositing a therapeutic device.

Still referring to Fig. 2, tube 34 may be formed from a flexible coiled wire, an extruded polymeric tube or from any one of numerous materials well-known in the art. When shaft 16 is flexed or bent, channel 36 may effectively lengthen along a curvilinear axis 31, causing a slight, relative movement between cable 30 and tube 34. As will be described next, instrument 10 is adapted such that end effector 14 and control 18 remain stationary even while this relative movement occurs and the user control 18 is released.

Therefore, control 18 is disengaged from end effector 14 in the first position such that the proximal end of cable 30 (also referred to as an actuating element) may move relative to the proximal end of shaft 16 while the distal end of cable 30 is stationary relative to end effector 14. This is also true for the other embodiments to be described next.

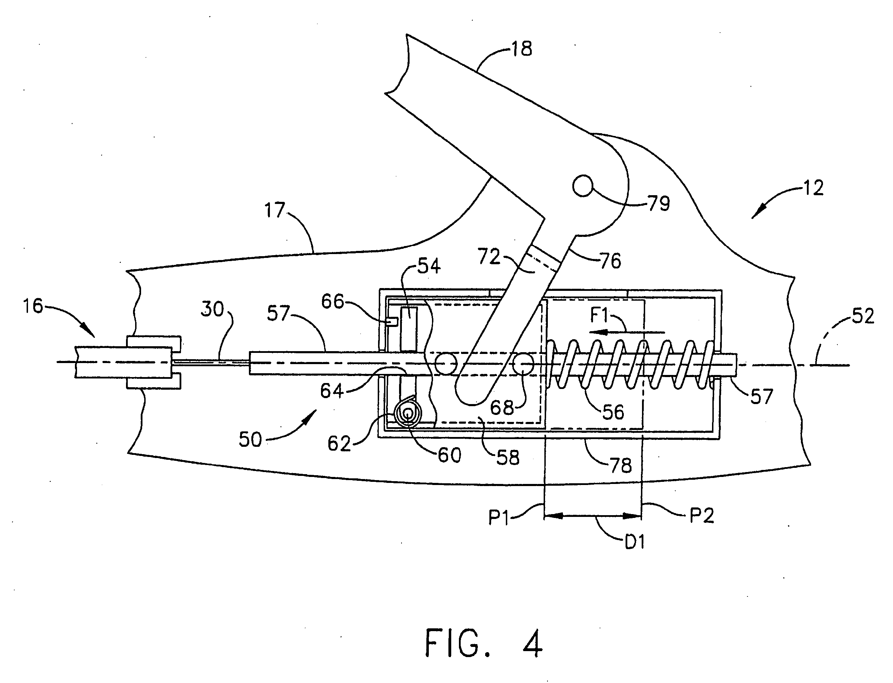

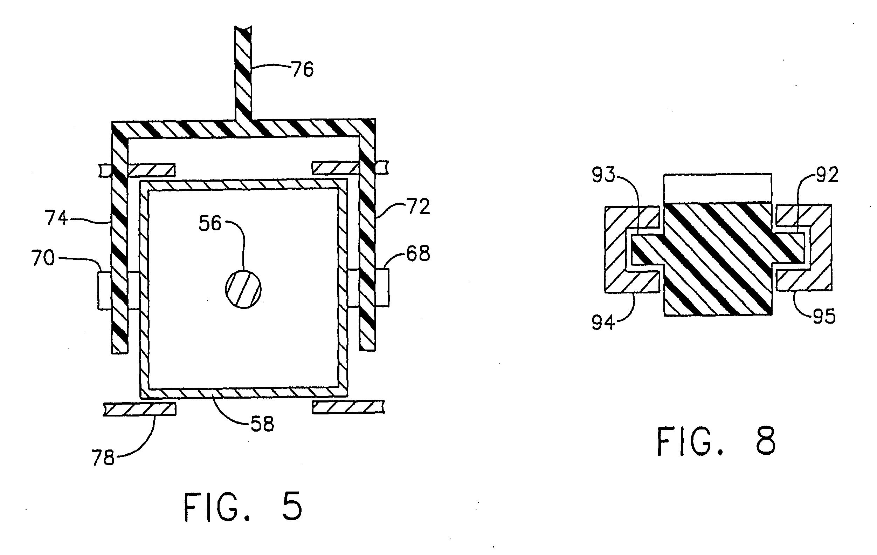

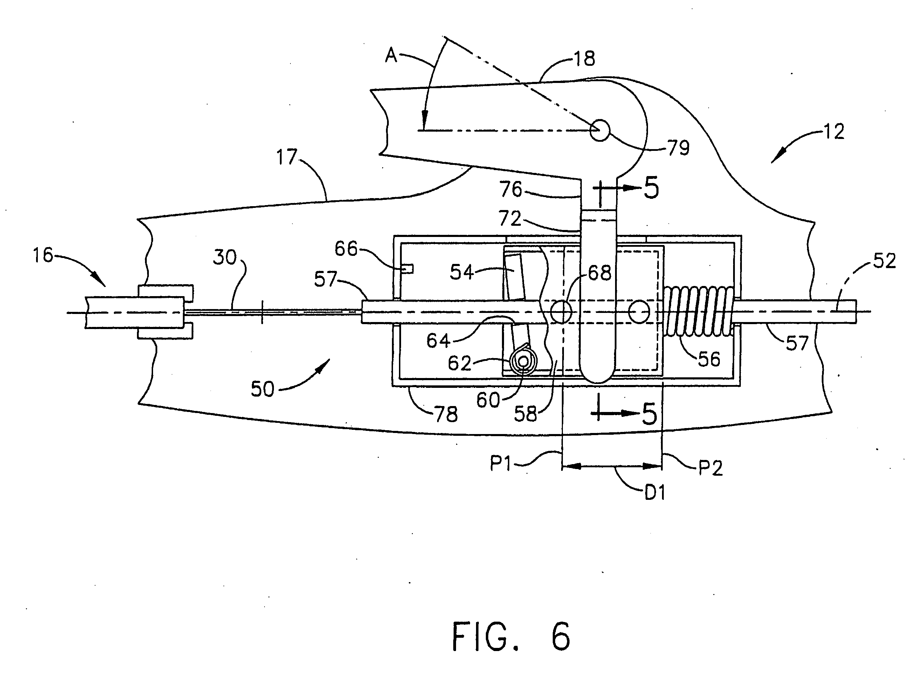

Figs. 4 and 6 are partial, cut-away views of handle 12 of instrument 10 and include a first embodiment of an engagement mechanism 50 mounted to grip 17. Fig. 5 is a cross-sectional view taken at line 5-5 of engagement mechanism 50 in Fig. 6. A user may operate control 18 between the first position (shown in Fig. 4) and the second position (shown in Fig. 6) to move engagement mechanism 50 along an axis 52. When control 18 is in the first position, cable 30 is disengaged from control 18. When a user moves control 18 from the first position, cable 30 engages with control 18 so that the user may operate end effector 14 shown in Fig. 2. A full actuation (indicated by "A") of control 18 moves engagement mechanism 50 along axis 52 a full stroke (indicated by "D1") from a first position endpoint (indicated by "P1" in Fig. 4) to a second position endpoint (indicated by "P2" in Fig. 4). Cable 30 is operatively connected to mechanism 50 such that cable 30 also moves a distance "D1" in the proximal direction.

It should be understood that the first position of user control 18 may refer to any of a number of positions corresponding to a small initial movement of engagement mechanism 50 sufficient to allow user control 18 to operatively engage with cable 30. During further movement of user control 18 beyond the first position (between the first and second positions) user control 18 and cable 30 remain operatively engaged. It should also be understood that the first and second positions of user control 18 correspond with the first and second configurations of end effector 14, respectively. Similarly, intermediate positions of user control 18 correspond with intermediate configurations of end effector 14.

Mechanism 50 includes a clutching element 54 for engaging control 18 to cable 30. Mechanism 50 may also include a spring 56 for applying a return force "F1" on cable 30 when clutching element 54 is engaged to cable 30. Spring 56 may be a compression spring formed from a coiled, stainless steel wire. In one embodiment, the return force may urge cable 30 to move distally, thereby urging end effector 14 to the opened configuration. Spring 56 may also urge user control 18 to be in the first position when user control 18 is released.

Clutching element 54 may be similar in form and operating principle to a device commonly used to hold a screen door spring/damper rod in an extended position. Clutching element 54 may be formed from a flat plate of a metal or rigid plastic and includes an aperture 64 that fits loosely over a drive rod 57 when clutching element 54 is approximately perpendicularly to drive rod 57. Clutching element 54 may be mounted to the inside of a box 58 at a pivot 60. In one embodiment, box 58 may be open on the distal (left) end face and closed on all other faces. Spring 56 may bear against the proximal (right) end face of box 58. A torque spring 62 may bias clutching element 54 to rotate about pivot 60. When mechanism 50 is in the first position, box 58 may be positioned such that a stop 66 attached to or unitarily formed with grip 17 holds clutching element 54 in an approximately perpendicular relationship with drive rod 57, allowing drive rod 57 to translate in the longitudinal direction independently of control 18.

Grip 17 may be formed from two half-shells joined together along a seam (not shown) to define a cavity that retains mechanism 50. A track 78 may extend from the inside of each half-shell to guide box 58 as it translates between the first and second positions. A pair of tines 72, 74 of a fork 76 extending from control 18 may operatively engage with posts 68, 70 extending from box 58, such that operation of control 18 between the first and second positions moves box 58 along axis 52 between endpoints P1, P2. In one embodiment, control 18 may be a lever that pivots about a lever pivot 79. When a user initially presses control 18, box 58 translates proximally (to the right) and clutching element 54 tilts, thereby locking onto drive rod 57 at aperture 64. Subsequent pressing of control 18 further drives box 58 proximally, thereby pulling cable 30 and moving end effector 14 from the opened configuration to the closed configuration. Release of control 18 at any point between the first and second positions allows spring 56 to return control 18 to the first position and end effector 14 to the opened configuration.

Instrument 10 and handle 12 of Figs. 4-6 have been described for applying a pulling or tensile force to cable 30. However, it should be understood that handle 12 may also be adapted to apply a pushing or compressive force to cable 30. Spring 56 may be a tension spring rather than a compression spring, and control 18 may be actuated in the opposite direction (clockwise about lever pivot 79) so that tines 72, 74 push guide box 58 from right to left, as viewed in Fig. 4.

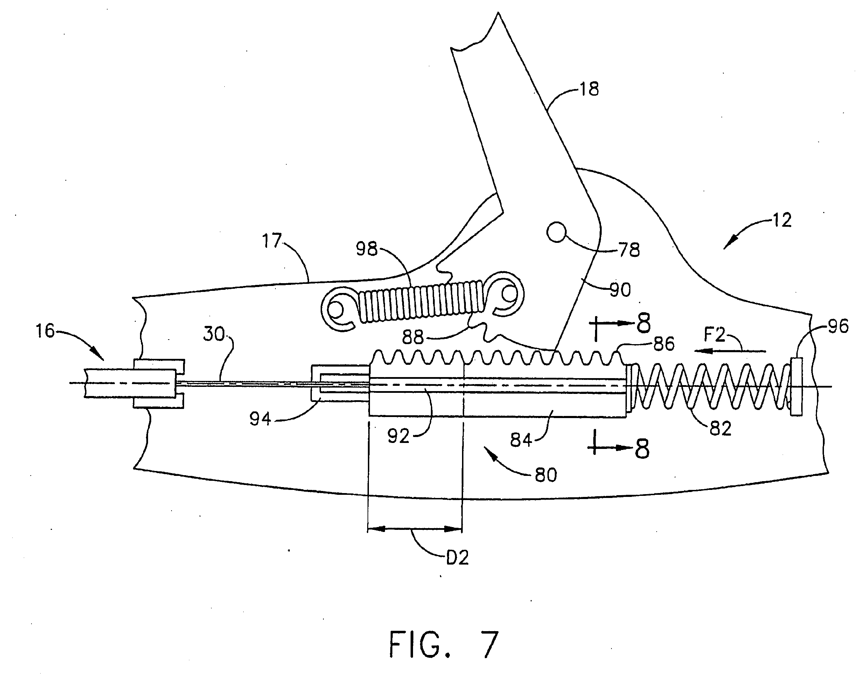

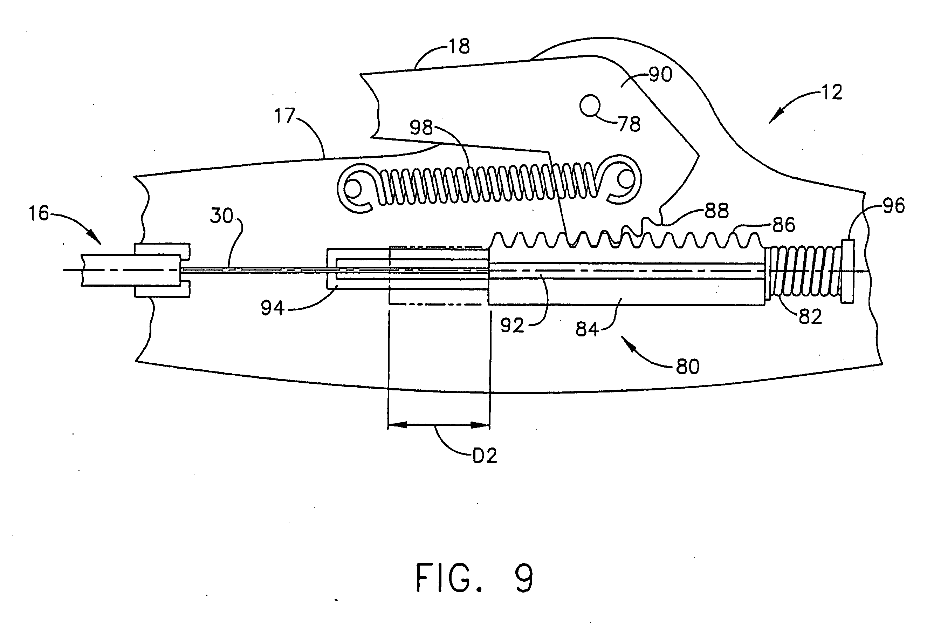

Figs. 7 and 9 are cut-away views of handle 12 of Fig. 1, showing an exemplary engagement mechanism not of the present invention, generally designated 80. Fig. 8 is a cross-sectional view taken at line 8-8 of mechanism 80 in Fig. 7. Mechanism 80 may include a clutching element 84 for engaging control 18 to cable 30 and a spring 82 for applying a return force "F2" on cable 30 in the distal direction. Spring 82 may be a compression spring formed from a stainless steel wire. Spring 82 may be positioned between the proximal end of clutching element 84 and a wall 96 depending from grip 17.

As may be seen in Fig. 7, clutching element 84 may be a rack gear segment (also referred to as a first gear) having a plurality of gear teeth 86. A pair of flanges 92, 93 may extend from the sides of clutching element 84 and may be slidably retained between a pair of tracks 94, 95 formed on grip 17. Cable 30 may be attached to the distal end of clutching element 84. Control 18 may include a gear sector 90 (also referred to as a second gear) having a plurality of gear teeth 88 adapted to engage with teeth 86 of clutching element 84 only when control 18 is moved from the first position. When control 18 is in the first position (released), cable 30 is disengaged from control 18 and spring 82 biases cable 30 in the distal direction. When a user operates control 18, teeth 88 may engage with teeth 86, such that element 84 and cable 30 move a distance "D2" in the proximal direction, thereby moving end effector 14 (Fig. 1) from the opened to the closed configuration. When a user releases control 18, a return spring 98 may move control 18 to the first position and spring 82 may move mechanism 80 distally, thereby moving end effector 14 to the opened configuration.

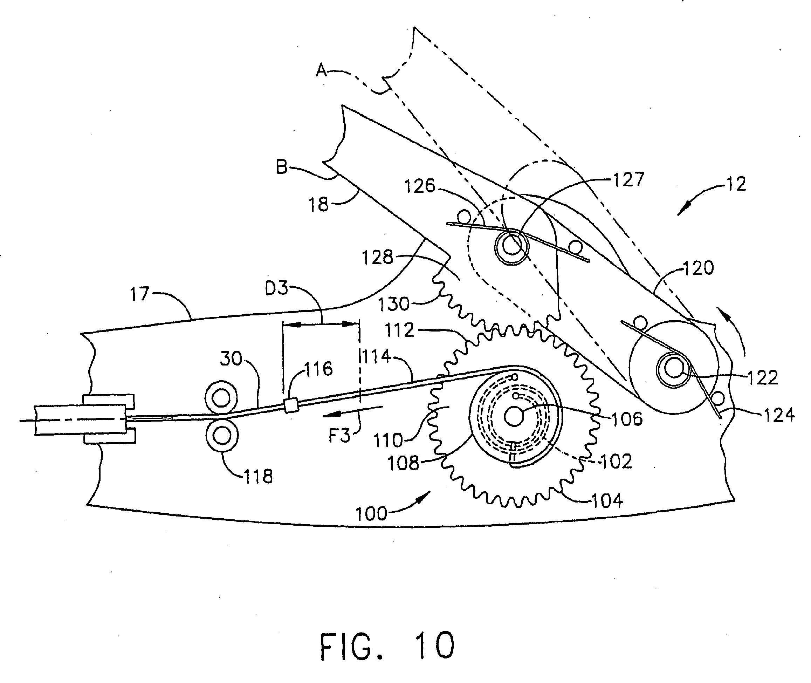

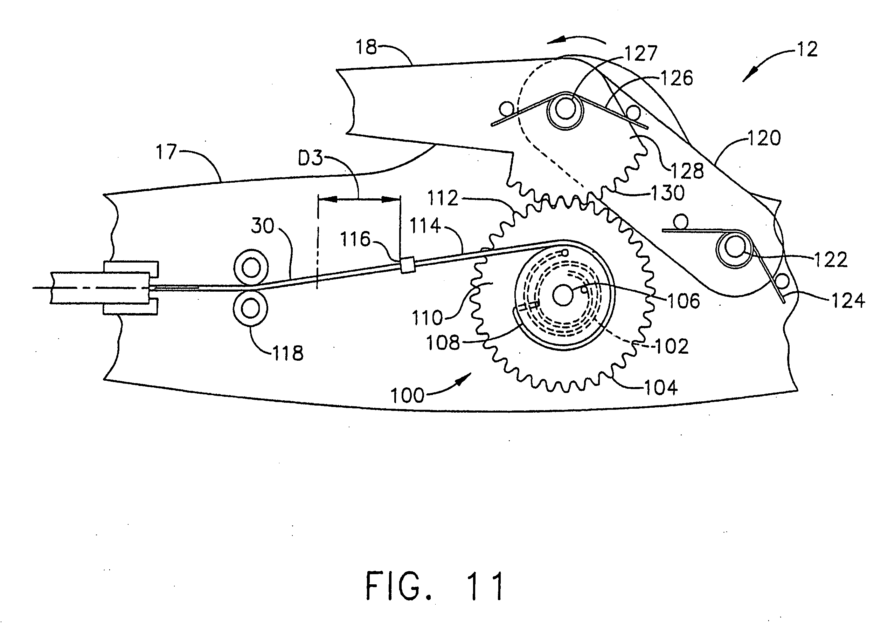

Figs. 10 and 11 are cut-away views of handle 12 of instrument 10 shown in Fig. 1, including another exemplary engagement mechanism not of the present invention, generally designated 100. Fig. 10 shows control 18 (in phantom) in a first position labeled "A" and an intermediate position labeled "B". Fig. 11 shows control 18 in a second position.

Engagement mechanism 100 may include a clutching element 104 for engaging control 18 to cable 30, and a spring 102 for applying a return force "F3" on cable 30 in the distal direction, thereby urging end effector 14 to move to the opened configuration when control 18 is released. Clutching element 104 may include a circular gear 110 (also referred to as a first gear) and a drum 108 concentrically and rotationally mounted on a pin 106 extending from the grip 17. Spring 102 may be formed from a spiral watch-spring and may be attached between grip 17 and clutching element 104 such that spring 102 applies a torque on clutching element 104 in a counter-clockwise direction (as viewed in Figs. 10 and 11) about a pin 106. The proximal end of cable 30 may be wrapped around drum 108 or, as shown, attached at a link 116 to a strap 114.

Control 18 may include a gear sector 128 (also referred to as a second gear) having a plurality of gear teeth 130 that may engage with a plurality of teeth 112 on clutching element 104 when control 18 is in the intermediate and second positions. Control 18 may be a lever that pivotally attaches to one end of a link 120 at a pivot 127. A first torsion spring 126 may bias control 18 and link 120 to be extended for the first and intermediate positions, as shown in Fig. 10. The opposite end of link 120 may pivotally attach to a pin 122 extending from grip 17. A second torsion spring 124 may bias link 120 to move to the first position, such that teeth 130 of gear sector 128 and teeth 112 of clutching element 104 are separated. First torsion spring 126 may be stiffer than second torsion spring 124 to insure that link 120 pivots about pin 122 before control 18 pivots about pivot 127 when the user actuates control 18.

When control 18 is in the first position (released), control 18 may be disengaged from cable 30, thereby allowing spring 102 to push cable 30 and bias end effector 14 to stay in the opened configuration. When a user presses control 18 to the intermediate position, control 18 may operatively engage cable 30. When the user presses control 18 to the second position, clutching mechanism 104 may rotate and take up strap 114 on drum 108, thereby pulling cable 30 a distance "D3" and closing end effector 14.

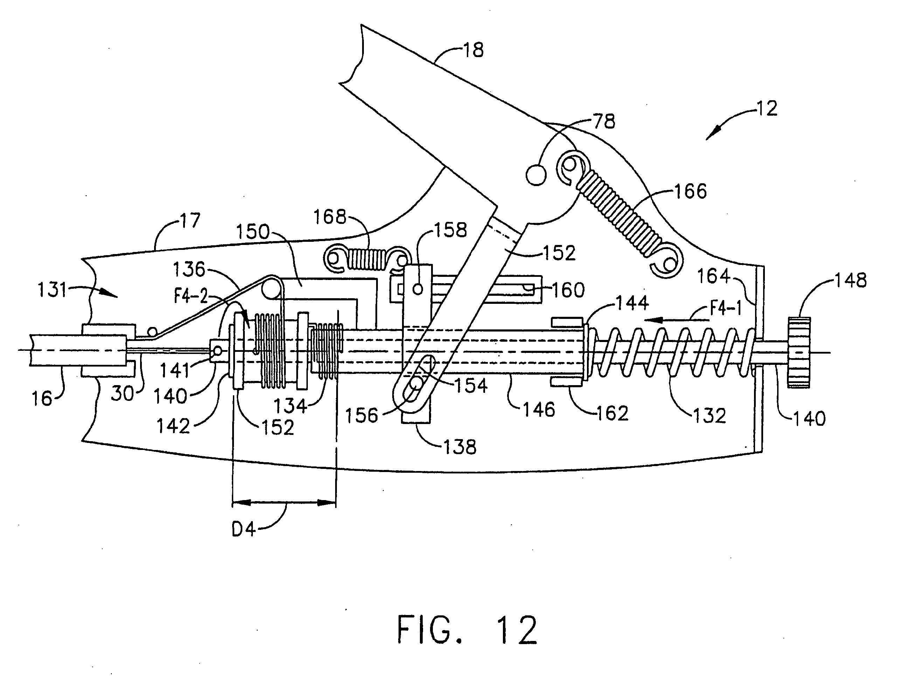

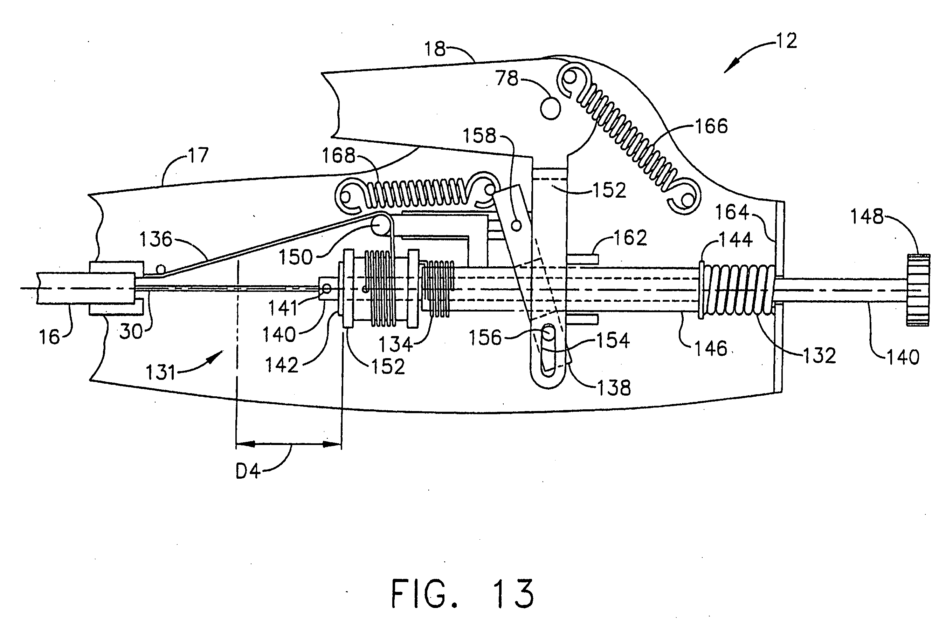

Figs. 12 and 13 are cut-away views of handle 12 of instrument 10 shown in Fig. 1, including yet another exemplary engagement mechanism not of the present invention, generally designated 131. Fig. 12 shows user control 18 in the first position and Fig. 13 shows user control 18 in the second position. Mechanism 131 may be adapted to actuate two cables that extend through shaft 16 and are operatively connected to the end effector 14. Cable 30 (also referred to as a first actuating element) may be actuated to operate a first function of the end effector, such as to open and/or close jaws 24, 26 (Fig. 2) as in the previous embodiments. A second actuating element 136 may be actuated to operate a second function of the end effector, such as deployment of a fastener, release of a mechanism contained in the jaw or extension of a probe. Second actuating element 136 may also be attached to linkage 28 such that cable 136 works antagonistically with cable 30 such that tension on cable 136 opens the end effector and tension on cable 30 closes the end effector.

Engagement mechanism 131 may include a clutching element 138 for engaging control 18 to cable 30, a first spring132 for applying a first force "F4-1" to cable 30, and a second spring 134 for applying a second force "F4-2" to cable 136. Clutching element 138 may be similar to clutching element 54 (the "screen door device") in Fig. 4. Clutching element 138 may be formed from a flat plate of metal or rigid plastic with an aperture (hidden) that slides freely over a drive cylinder 146 when clutching element 138 is in a first orientation (here shown to be approximately perpendicular) with respect to drive cylinder 146. When clutching element 138 is tilted to a second orientation as shown in Fig. 13, it locks onto drive cylinder 146 at the aperture such that control 18 may move drive cylinder 146 in the proximal direction, thereby closing end effector 14.

A fork 152 may extend from control 18 and may be similarly configured as fork 76 shown in Fig. 5. Fork 152 may include slots 154 that retain a pair of lower pivots 156 extending from the sides of clutching element 154. A pair of upper pivots 158 may extend from the sides of clutching element 138 and may be slidably retained in a pair of tracks 160 formed on opposing sides of grip 17. When a user presses control 18, fork 152 may rotate about pivot 78, tilt clutching element 138 to lock onto drive cylinder 146 and force drive cylinder 146 in the proximal direction, thereby pulling cable 30. When the user releases control 18, a return spring 166 may move control 18 to the first position, a clutch spring 168 may return clutching element 138 to the second (upright) orientation and first biasing element 132 may move mechanism 131 and cable 30 distally.

User control 18 may be disengaged from cable 30 when in the first position (released) and engaged with cable 30 between the first and second positions. A control knob 148, however, may always be engaged with second actuating element 136. Knob 148 may be attached to the proximal end of a drive shaft 140 that may rotate freely inside of drive cylinder 146. Drive cylinder 146, however, may be configured on its outer surface to be constrained from rotating by features 162 extending from grip 17. A reel 152 may be rotatably attached to the distal end of drive shaft 160. Drive cylinder 146 and reel 152 may be retained on drive shaft 160 between a pair of snap rings 142, 144. The proximal end of cable 136 may be wrapped around reel 152. An arm 150 may extend from drive cylinder 146 and may be positioned to guide cable 136 onto reel 152, such that a user may turn knob 148 to take-up and let-out cable 136 regardless of how control 18 and mechanism 131 are longitudinally positioned. The proximal end of cable 30 may be rotatably retained on the distal end of drive shaft 140 by a ball connector 141 such that rotation of drive shaft 140 does not twist cable 30.

First biasing element 132 may be a coiled steel wire compression spring assembled over shaft 140 and positioned between the proximal end of drive cylinder 146 and a wall 164 of grip 17. Second biasing element 134 may be a steel wire torsion spring assembled over the distal end of drive cylinder 146 and attached between reel 152 and drive cylinder 146 to provide force F4-2 on cable 136. Depending on how cable 136 is routed through grip 17, cable 136 may wrap or unwrap a small amount from reel 152 as required while mechanism 131 translates between the first and second positions.

The engagement mechanisms described and claimed herein may be adapted to any one of a number of medical instruments, including medical instruments having end effectors adapted to grasp or clamp tissue, to hold a surgical needle, to apply a fastener, to retract tissue, to cut tissue and to apply energy. In addition, although shaft 16 of instrument 10 has been described herein as being generally elongated, tubular, flexible and including an enclosed channel for retaining the actuating element, the shaft may also be relatively short, non-tubular, frame-like, relatively rigid and the channel may not be enclosed, but rather be defined by features on the shaft for guiding, retaining and/or housing an actuating element that operatively connects a user control to an end effector. In general, the engagement mechanism described herein may be adapted to medical instruments for which there is unintentional, relative movement between the shaft and the actuating element, such as during manipulation of the instrument. The engagement mechanisms described herein may also be incorporated into such medical instruments in order to preclude the need to custom assemble each instrument due to dimensional variations of particular instrument components.

Accordingly, it should be understood that although a medical instrument has been shown and described with respect to certain embodiments, modifications may occur to those skilled in the art. The medical instrument includes such modifications and is limited only by the scope of the claims.

## Claims

1. A medical instrument (10) comprising:
a shaft (16) having a channel (36) extending therethrough, said shaft (16) including a proximal end (20) and a distal end (22);
an actuating element (30) extending through said channel (36), said actuating element (30) having a proximal and a distal end;
an end effector (14) disposed on said distal end (22) of said shaft (16) and operatively connected to said distal end of said actuating element (30), wherein said actuating element (30) is adapted to move said end effector (14) between a first configuration and a second configuration;
a control (18) moveable between a first position corresponding to said first configuration and a second position corresponding to said second configuration, wherein said control (18) is operatively disconnected from said actuating element (30) when said control (18) is in said first position; and
an engagement mechanism (50) including a pivotable clutching element (54) adapted to releasably engage said control (18) to said actuating element (30) when said control (18) is moved from said first position;
wherein said clutching element (54) is formed from a rigid material having an aperture (64) therethrough;
**characterised in that** said engagement mechanism (50) further comprises a drive element (57) attached to said proximal end of said actuating element (30) and inserted through said aperture (64), whereby moving said control (18) from said first position pivots said clutching element (54) with respect to said drive element (57) such that said clutching element (54) locks onto said drive element (57) at said aperture (64), wherein further movement of said control moves said drive element which in turn drives said actuating element to move said end effector into the second configuration.

2. The medical instrument of claim 1 wherein said shaft (16) and said actuating element (30) are flexible.

3. The medical instrument of claim 1 wherein said shaft (16) and said end effector (14) are sized for insertion into a working channel of an endoscope.

4. The medical instrument of claim 1 wherein said end effector (14) is adapted to grasp tissue, clamp tissue, hold a needle, apply a fastener, apply a clip, retract tissue, cut tissue or apply an energy to tissue.

5. The medical instrument (10) of claim 1, wherein the engagement mechanism (50) includes a spring element (56) adapted to apply a return force to said actuating element (30) such that said end effector (14) is biased to said first position.

6. The medical instrument of any preceding claim further comprising a handle (12) attached to said proximal (20) end of said shaft (16).

7. The instrument of claim 1, comprising a pivot (60) about which said clutching member rotates.

8. The instrument of claim 7, comprising a guide box (58) in which said clutching member and pivot are housed, said guide box being slidably received in a housing of said medical instrument.

9. The instrument of claim 8, wherein said guide box is slidably received on a track (78) of said housing of said medical instrument.

10. The instrument of claim 9, wherein said control is operable to move said guide box to move said clutching member from said first position to said second position.

11. The instrument of claim 10, comprising a fork (76) having a tine (72, 74) extending from said control to engage with a post (68, 70) extending from said guide box.

## Patentansprüche

1. Medizinisches Instrument (10), das Folgendes umfasst:
einen Schaft (16) mit einem sich durch diesen hindurch erstreckenden Kanal (36), wobei der Schaft (16) ein proximales Ende (20) und ein distales Ende (22) aufweist;
ein Betätigungselement (30), das sich durch den Kanal (36) erstreckt, wobei das Betätigungselement (30) ein proximales und ein distales Ende aufweist;
ein Greiforgan (14), das am distalen Ende (22) des Schafts (16) angeordnet ist und mit dem distalen Ende des Betätigungselenents (30) in Wirkverbindung steht,
worin das Betätigungselement (30) das Greiforgan (14) zwischen einer ersten Konfiguration und einer zweiten Konfiguration bewegen kann;
eine Steuerung (18), die zwischen einer ersten Stellung, die der ersten Konfiguration entspricht, und einer zweiten Stellung, die der zweiten Konfiguration entspricht, bewegt werden kann, worin die Steuerung (18) aus der Wirkverbindung mit dem Betätigungselement (30) gelöst wird, wenn sich die Steuerung (18) in der ersten Stellung befindet; und
einen Eingriffsmechanismus (50) mit einem schwenkbaren Kupplungselement (54), das die Steuerung (18) in lösbaren Eingriff mit dem Betätigungselement (30) bringen kann, wenn die Steuerung (18) aus der ersten Stellung bewegt wird;
worin das Kupplungselement (54) aus einem starren Material mit einer Öffnung (64) durch es hindurch geformt ist;
**dadurch gekennzeichnet, dass** der Eingriffsmechanismus (50) ferner ein Antriebselement (57) umfasst, das am proximalen Ende des Betatigungselements (30) befestigt und durch die Öffnung (64) eingeführt ist, wodurch eine Bewegung der Steuerung (18) aus der ersten Stellung das Kupplungselement (54) relativ zum Antriebselement (57) schwenkt, so dass das Kupplungselement (54) auf dem Antriebselement (57) an der Öffnung (64) arretiert, worin eine weitere Bewegung der Steuerung das Antriebselement bewegt, welches wiederum das Betätigungselement antreibt, um das Greiforgan in die zweite Konfiguration zu bewegen.

2. Medizinisches Instrument nach Anspruch 1, worin der Schaft (16) und das Betätigungselement. (30) flexibel sind.

3. Medizinisches Instrument nach Anspruch 1, worin der Schaft (16) und das Greiforgan (14) eine solche Größe aufweisen, dass sie in einen Arbeitskanal eines Endoskops eingeführt werden können.

4. Medizinisches Instrument nach Anspruch 1, worin das Greiforgan (14) Gewebe greifen, Gewebe klammern, eine Nadel halten, ein Befestigungsglied anbringen, eine Klammer setzen, Gewebe zurückziehen, Gewebe schneiden oder eine Energie auf Gewebe aufbringen kann.

5. Medizinisches Instrument (10) nach Anspruch 1, worin der Eingriffsmechanismus (50) ein Federelement (56) aufweist, das eine Rückführungskraft auf das Betätigungselement (30) ausüben kann, so dass das Greiforgan (14) in die erste Stellung vorgespannt wird.

6. Medizinisches Instrument nach einem der vorhergehenden Ansprüche, das einen am proximalen Ende (20) des Schafts (16) befestigten Handgriff (12) umfasst.

7. Instrument nach Anspruch 1, das einen Drehpunkt (60) umfasst, um den sich das Kupplungselement dreht.

8. Instrument nach Anspruch 7, das eine Führungsbox (58) umfasst, in der das Kupplungselement und der Drehpunkt untergebracht sind, wobei die Führungsbox gleitend im Gehäuse des medizinischen Instruments aufgenommen ist.

9. Instrument nach Anspruch 8, worin die Führungsbox gleitend auf einer Bahn (78) des Gehäuses des medizinischen Instruments aufgenommen ist.

10. Instrument nach Anspruch 9, worin die Steuerung zur Bewegung des Kupplungselements aus der ersten Stellung in die zweite Stellung die Führungsbox bewegen kann.

11. Instrument nach Anspruch 10, das eine Gabel (76) mit Zinken (72, 74), die sich zum Eingriff mit einem von der Führungsbox ausgehenden Pfosten (68, 70) von der Steuerung aus erstrecken, umfasst.

## Revendications

1. Instrument médical (10) comprenant :
une tige (16) ayant un canal (36) s'étendant à travers elle, ladite tige (16) comportant une extrémité proximale (20) et une extrémité distale (22) ;
un élément d'actionnement (30) s'étendant à travers ledit canal (36), ledit élément d'actionnement (30) ayant une extrémité proximale et une extrémité distale ;
un effecteur terminal (14) disposé sur ladite extrémité distale (22) de ladite tige (16) et connecté fonctionnellement à ladite extrémité distale dudit élément d'actionnement (30), ledit élément d'actionnement (30) étant adapté de manière à déplacer ledit effecteur terminal (14) entre une première configuration et une deuxième configuration ;
une commande (18) déplaçable entre une première position correspondant à ladite première configuration et une deuxième position correspondant à ladite deuxième configuration, ladite commande (18) étant déconnectée fonctionnellement dudit élément d'actionnement (30) lorsque ladite commande (18) est dans ladite première position ; et
un mécanisme d'engagement (50) incluant un élément d'embrayage pivotant (54) prévu de manière à engager de manière libérable ladite commande (18) avec ledit élément d'actionnement (30) lorsque ladite commande (18) est déplacée depuis ladite première position ;
ledit élément d'embrayage (54) étant formé à partir d'un matériau rigide ayant une ouverture (64) à travers lui ;
**caractérisé en ce que** ledit mécanisme d'engagement (50) comprend en outre un élément d'entraînement (57) attaché à ladite extrémité proximale dudit élément d'actionnement (30) et inséré à travers ladite ouverture (64), le déplacement de ladite commande (18) depuis ladite première position faisant pivoter ledit élément d'embrayage (54) par rapport audit élément d'entraînement (57) de telle sorte que ledit élément d'embrayage (54) se verrouille sur ledit élément d'entraînement (57) au niveau de ladite ouverture (64), un déplacement supplémentaire de ladite commande déplaçant ledit élément d'entraînement, qui à son tour entraîne ledit élément d'actionnement afin qu'il déplace ledit effecteur terminal dans la deuxième configuration.

2. Instrument médical selon la revendication 1, dans lequel ladite tige (16) et ledit élément d'actionnement (30) sont flexibles.

3. Instrument médical selon la revendication 1, dans lequel ladite tige (16) et ledit effecteur terminal (14) sont dimensionnés de manière à être insérés dans un canal de travail d'un endoscope.

4. Instrument médical selon la revendication 1, dans lequel ledit effecteur terminal (14) est adapté de manière à saisir des tissus, à serrer des tissus, à retenir une aiguille, à appliquer une agrafe, à appliquer une pince, à retirer des tissus, à couper des tissus ou à appliquer de l'énergie à des tissus.

5. Instrument médical (10) selon la revendication 1, dans lequel le mécanisme d'engagement (50) comporte un élément de ressort (56) adapté de manière à appliquer une force de retour audit élément d'actionnement (30) de telle sorte que ledit effecteur terminal (14) soit sollicité dans ladite première position.

6. Instrument médical selon l'une quelconque des revendications précédentes, comprenant en outre une poignée (12) attachée à ladite extrémité proximale (20) de ladite tige (16).

7. Instrument selon la revendication 1, comprenant un pivot (60) autour duquel tourne ledit organe d'embrayage.

8. Instrument selon la revendication 7, comprenant une boite de guidage (58) dans laquelle sont logés ledit organe d'embrayage et ledit pivot, ladite boîte de guidage pouvant être reçue de manière coulissante dans un logement dudit instrument médical.

9. Instrument selon la revendication 8, dans lequel ladite boîte de guidage est reçue de manière coulissante sur une glissière (78) dudit logement dudit instrument médical.

10. Instrument selon la revendication 9, dans lequel ladite commande peut fonctionner de manière à déplacer ladite boite de guidage de manière à déplacer ledit organe d'embrayage depuis ladite première position dans ladite deuxième position.

11. Instrument selon la revendication 10, comprenant une fourche (76) ayant une dent (72, 74) s'étendant depuis ladite commande afin de s'engager avec un ergot (68, 70) s'étendant depuis ladite boite de guidage.
